(19) Europäisches Patentamt
European Patent Office
Office européen des brevets

(11) Veröffentlichungsnummer: **0 169 809**
**B1**

(12) # EUROPÄISCHE PATENTSCHRIFT

(45) Veröffentlichungstag der Patentschrift: **25.11.87**

(51) Int. Cl.⁴: **C 07 C 149/273,** C 07 C 148/00

(21) Anmeldenummer: **85810329.4**

(22) Anmeldetag: **19.07.85**

(54) **Verfahren zur Herstellung von phenolischen Thiocarbonsäureestern.**

(30) Priorität: **25.07.84 CH 3599/84**

(43) Veröffentlichungstag der Anmeldung: **29.01.86 Patentblatt 86/5**

(45) Bekanntmachung des Hinweises auf die Patenterteilung: **25.11.87 Patentblatt 87/48**

(84) Benannte Vertragsstaaten: **DE FR GB IT**

(56) Entgegenhaltungen:
**EP-A-0 059 168**
**DE-A-2 059 916**
**DE-A-2 838 273**
**DE-A-2 936 803**
**FR-A-1 560 259**
**US-A-3 553 270**
**US-A-3 810 869**
**US-A-3 832 328**

(73) Patentinhaber: **CIBA- GEIGY AG, Klybeckstrasse 141, CH- 4002 Basel (CH)**

(72) Erfinder: **Rosenberger, Siegfried, Im Gehracker 11, CH- 4125 Riehen (CH)**
Erfinder: **Stegmann, Werner, Dr., Unter der Sonnhalde 9, CH- 4410 Liestal (CH)**

**EP 0 169 809 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues Einstufenverfahren zur Herstellung phenolischer Thiocarbonsäureester.

Die Herstellung phenolischer Thiocarbonsäureester in zwei Stufen, entweder über die Mannichbase oder durch Umsetzung von entsprechenden Phenolen mit Merceptosäuren und Formaldehyd und anschliessender Veresterung der erhaltenen Säuren, ist bekannt. Sie wird z. B. im US-Patent 3 832 328 und, was die Mannichreaktion betrifft, auch in EP-Patentanmeldung 59 168 beschrieben. Diese Zweistufenverfahren sind jedoch aufwendig und die Ausbeute sowie die Reinheit der erhaltenen Produkte sind unbefriedigend. US-Patent 3 553 270 beschreibt ein Einstufenverfahren zur Herstellung von phenolischen Thioethern, durch Umsetzung des entsprechenden Phenols mit Formaldehyd und einem Mercaptan in Gegenwart einer starken Base, wie Trimethylamin oder insbesondere Alkalimetallhydroxyde, als Katalysator. Versuche, nach dieser Methode auch phenolische Thiocarbonsäureester herzustellen, sind gescheitert. Es wurde aber gefunden, dass bei Verwendung primärer oder sekundärer Amine als Katalysatoren, phenolische Thiocarbonsäureester überraschenderweise in hoher Reinheit und Ausbeute erhalten werden.

Die vorliegende Erfindung betrifft demnach ein Verfahren zur Herstellung von Verbindungen der Formel I

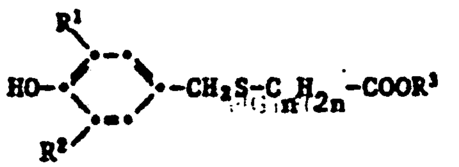

(I),

worin $R^1$ und $R^2$ unabhängig voneinander tert.-Butyl, $R_3$ $C_1$-$C_{20}$ Alkyl oder durch -O- oder -S- unterbrochenes $C_2$-$C_{20}$ Alkyl und n die Zahl 1 oder 2 bedeuten, durch Umsetzung eines Phenols der Formel II

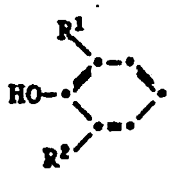

(II)

mit Formaldehyd und einem Mercaptan der Formel III

$R^3OOC$-$C_nH_{2n}$-$SH$ (III),

worin $R^1$, $R^2$, $R^3$ und n die oben angegebene Bedeutung haben, bei Temperaturen zwischen 20 und 200°C in Gegenwart eines Katalysators, dadurch gekennzeichnet, dass der Katalysator ein primäres oder sekundäres Amin der Formel IV

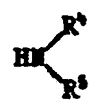

(IV)

ist, worin $R^4$ und $R^5$ unabhängig voneinander $C_2$-$C_{20}$ Alkyl oder Hydroxyalkyl, $C_5$-$C_7$ Cycloalkyl, Phenyl oder Benzyl bedeuten, und $R^5$ zusätzlich auch Wasserstoff sein kann.

$R^3$ ist als $C_1$-$C_{20}$ Alkyl z. B. Methyl, Ethyl, Propyl, Isopropyl, n-Butyl, sek.-Butyl, tert.-Butyl, Pentyl, Hexyl, n-Octyl, 2-Ethylhexyl, 1,1,3,3-Tetramethylbutyl, Nonyl, Decyl, Dodecyl, Tridecyl, Hexadecyl, Octadecyl oder Eicosyl. Bevorzugt ist $R^3$ 2-Ethylhexyl oder iso-Tridecyl (Tridecylisomerengemisch).

Beispiele für $R^3$ als durch -O- oder -S- unterbrochenes $C_2$-$C_{20}$ Alkyl sind: Methoxymethyl, 2-Ethoxyethyl, 2-n-Butoxyethyl, 3-n-Butoxy-ethyl, 2-Octoxyethyl, 2-Hexadecyloxyethyl, 2-Ethoxymethyl, Butoxymethyl, Methoxypropyl, Ethoxypropyl, 3-Thiaheptyl oder 3-Thia-5-methylhexyl.

n ist bevorzugt 1.

$R^4$ und $R^5$ sind als $C_2$-$C_{20}$ Alkyl z. B. Ethyl, Propyl, Isopropyl, n-Butyl, sek.-Butyl, tert.-Butyl, Pentyl, Hexyl, Octyl, Decyl, Dodecyl, Hexadecyl, Octadecyl oder Eicosyl und als $C_2$-$C_{20}$ Hydroxyalkyl, 2-Hydroxyethyl, 3-Hydroxypropyl, 3-Hydroxybutyl, 3-Hydroxyoctyl, 2-Hydroxybutyl, 3-Hydroxypentyl.

Als $C_5$-$C_7$ Cycloalkyl bedeuten $R^4$ und $R^5$ z. B. Cyclopentyl, Cycloheptyl und insbesondere Cyclohexyl.

Bevorzugt sind $R^4$ und $R^5$ $C_2$-$C_8$ Alkyl und $R^5$ zusätzlich auch Wasserstoff. Ganz besonders bevorzugt sind $R^4$ und $R^5$ gleich und bedeuten insbesondere n-Butyl.

Das erfindungsgemässe Verfahren wird besonders zweckmässig durch Umsetzung von 1 Mol des Phenols der Formel II mit 1,0 bis 2,0 Mol Formaldehyd und 0,9 bis 1,3 Mol eines Mercaptans der Formel III in Gegenwsrt von mindestens 0,5 Mol %, bezogen auf das Phenol der Formel II, des Katalysators der Formel IV durchgeführt.

Vorzugsweise werden 1 bis 15 Mol %, insbesondere aber 2,5 bis 10 Mol %, bezogen auf das Phenol, des Katalysators eingesetzt.

Die Reaktion kann mit oder ohne Lösungsmittel durchgeführt werden. Geeignete Lösungsmittel sind solche, die ein gewisses Lösungsvermögen gegenüber den Reaktanden haben, aber unter den Reaktionsbedingungen im wesentlichen inert sind. Beispiele dafür sind Kohlenwasserstoffe wie Toluol, Xylol, Octan und β-Terpen, Ether wie Dioxan, Diethylether, Dimethylether von Ethylenglykol, Tetrahydrofurhan u.s.w. Auch chlorierte Kohlenwasserstoffe, wie Tetrachlorkohlenstoff, Chloroform, Trichlorethan, Perchlorethylene können vorteilhaft eingesetzt werden. Andere geeignete Lösungsmittel sind z. B. Dimethylformamid, Dimethylacetamid oder Dimethylsulfoxid. Auch primäre oder sekundäre Alkohole mit 3 bis 6 C-Atomen, wie Isopropanol, sek.-Butylalkohol, tert.-Butylalkohol, tert.-Amylalkohol und Hexylalkohol können für eine erfolgreiche Durchführung des erfindungsgemässen Verfahrens empfohlen werden.

Das Formaldehyd kann als wässrige Lösung, insbesondere als ca. 35 %-ige wässrige Lösung, oder in Form von Paraformaldehyd vorliegen. Verwendet man Paraformaldehyd, dann ist es nötig die 5- bis 20-fache, bevorzugt die 8- bis 12-fache Menge, im Gewicht, bezogen auf das Paraformaldehyd, eines Depolymerisators, wie z. B. Dimethylformamid, Dimethylacetamid oder Dimethylsulfoxid, zuzugeben.

Die Reaktionstemperatur variiert je nach Reaktand. Geeignete Temperaturen liegen zwischen 20 und 200°C. Ein bevorzugter Temperaturbereich ist zwischen 80 und 140°C. In der Praxis erweist es sich als zweckmässig das Verfahren bei der Siedetemperatur des Reaktionsgemisches durchzuführen. Demnach ist der Temperaturbereich zwischen 80°C und der Siedetemperatur des Reaktionsgemisches besonders bevorzugt.

Die Reaktionsdauer ist ebenfalls von den Reaktanden abhängig; im allgemeinen ist die Reaktion nach 4 bis 8 Stunden beendet. Die Isolierung des Endproduktes geschieht durch übliches Abtrennen der flüchtigen Anteile, z. B. durch Abdestillieren im Vakuum, oder durch Herauslösen von gegebenenfalls unflüchtigen aminischen Katalysatorresten mit verdünnter Säure, z. B. verdünnter Salz- oder Schwefelsäure. Wegen dem hohen Reinheitsgrad der erhaltenen Produkte ist keine zusätliche Reinigung erforderlich.

Die Verbindungen der Formel I sind wertvolle Stabilisatoren für organische Materialien, die der Zersetzung durch Licht, Wärme oder Sauerstoff unterworfen sind, vorzugsweise für Elastomere und Schmiermittel.

Die folgenden Beispiele erläutern die Erfindung ohne sie einzuschränken.

**Beispiel 1:**

Ein Gemisch aus 206 g 2,6-Di-tert.-butylphenol, 45 g Paraformaldehyd, 204 g Thioglykolsäure-2-ethyl-n-hexylester, 7,6 g Di-n-butylamin und 150 ml Dimethylformamid wird unter Rückfluss und Stickstoffzufuhr 3 bis 4 Stunden bei ca. 120°C gerührt. Anschliessend werden die flüchtigen Anteile (Rest-Aldehyd, Di-n-butylamin und Dimethylformamid) durch Abdestillieren im Vakuum vollständig entfernt. Man erhält so 415 g (98 % d.Th.) 3,5-Di-tert.-butyl-4-hydroxybenzylmercapto-essigsäure-2-ethyl-n-herylester in Form eines viskosen blassgelblichen Öles, welches beim Stehen in der Kälte kristallin erstarrt (Smp. 20°C). Das Produkt erweist sich, ohne weitere Reinigungsschritte, nach Kontrolle mit Dünnschichtchromatographie, NMR und Phenoltitration, als sehr rein (Gehalt > 98 %).

**Beispiele 2-4:**

Ersetzt man im Beispiel 1 den Thioglykolsäure-2-ethyl-n-hexylester jeweils durch die äquivalente Menge eines der homologen Thioglykolester der Formel

$$HS-CH_2-\overset{\displaystyle O}{\overset{\|}{C}}O-R^3 ,$$

worin $R^3$ die in der nachstehenden Tabelle angebene Bedeutung hat, so erhält man bei analoger Arbeitsweise die entsprechenden homologen Phenole in praktisch der gleichen hohen Reinheit und Ausbeute.

| Beispiel | $R^3$ | Aspekt |
|---|---|---|
| 2 | $n\text{-}C_8H_{17}$ | kristallin, Smp. 35°C |
| 3 | $n\text{-}C_{12}H_{25}$ | viskoses Öl |
| 4 | $iso\text{-}C_{13}H_{27}$ | viskoses Öl |

**Beispiel 5:**

Verwendet man in Beispiel 1 anstelle von Dimethylformamid die äquivalente Menge Dimethylacetamid (als Depolymerisator des Paraformaldehyds), so erhält man bei sonst gleicher Arbeitsweise das Produkt von Beispiel 1 in ähnlicher Qualität und Ausbeute.

**Beispiel 6:**

Verwendet man in Beispiel 1 anstelle von Paraformaldehyd die aliquote Menge Formaldehyd in Form einer 35 %-igen wässrigen Lösung unter Verzicht suf den Dimethylformamid-Zusatz, so erhält man innert 7 bis 8 Stunden Reaktionszeit bei etwa 100°C, bei sonst gleicher Arbeitsweise, das Produkt von Beispiel 1 in ähnlicher Qualität und Ausbeute.

**Beispiel 7:**

Ersetzt man im Beispiel 1 das Di-n-butylamin durch die äquivalente Menge n-Octylamin, so erhält man bei analoger Arbeitsweise das Produkt von Beispiel 1 in ähnlicher Ausbeute und Reinheit.

**Patentansprüche**

1. Verfahren zur Herstellung von Verbindungen der Formel I

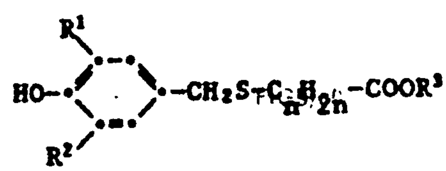

(I),

worin $R^1$ und $R^2$ unabhängig voneinander tert.-Butyl, $R^3$ $C_1$-$C_{20}$ Alkyl oder durch -O- oder -S- unterbrochenes $C_2$-$C_{20}$ Alkyl und n die Zahl 1 oder 2 bedeuten, durch Umsetzung eines Phenols der Formel II

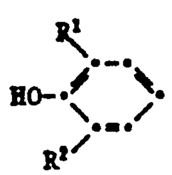

(II)

mit Formaldehyd und einem Mercaptan der Formel III

$R^3OOC\text{-}C_nH_{2n}\text{-}SH$ (III)

worin $R^1$, $R^2$, $R^3$ und n die oben angegebene Bedeutung haben, bei Temperaturen zwischen 20 und 200°C in Gegenwart eines Katalysators, dadurch gekennzeichnet, dass der Katalysator ein primäres oder sekundäres Amin der Formel IV

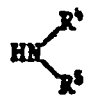

(IV)

ist, worin $R^4$ und $R^5$ unabhängig voneinander $C_2$-$C_{20}$ Alkyl oder Hydroxyalkyl, $C_5$-$C_7$ Cycloalkyl, Phenyl oder Benzyl bedeuten, und $R^5$ zusätzlich auch Wasserstoff sein kann.

2. Verfahren gemäss Anspruch 1, zur Herstellung von Verbindungen der Formel I, worin $R^1$ und $R^2$ unabhängig voneinander tert.-Butyl und $R^3$ $C_4$-$C_{13}$ Alkyl bedeuten.

3. Verfahren gemäss Anspruch 2, zur Herstellung von Verbindungen der Formel I, worin n die Zahl 1 bedeutet.

4. Verfahren gemäss Anspruch 1, zur Herstellung von 3,5-Di-tert.-butyl-4-hydroxybenzylmercapto-essigsäure-2-ethyl-n-hexylester.

5. Verfahren gemäss Anspruch 1, zur Herstellung von 3,5-Di-tert.-butyl-4-hydroxybenzyl-mercaptoessigsäure-isotridecylester.

6. Verfahren gemäss Anspruch 1, dadurch gekennzeichnet, dass 1 Mol des Phenols der Formel II mit 1,0 bis 2,0 Mol Formaldehyd und 0,9 bis 1,3 Mol eines Mercaptans der Formel III in Gegenwart von mindestens 0,5 Mol %, bezogen auf das Phenol der Formel II, des Katalysators der Formel IV umgesetzt wird.

7. Verfahren gemäss Anspruch 1, dadurch gekennzeichnet, dass ein Katalysator der Formel IV, worin $R^4$ und $R^5$ $C_2$-$C_8$ Alkyl bedeuten und $R^5$ zusätzlich auch Wasserstoff sein kann, eingesetzt wird.

8. Verfahren gemäss Anspruch 1, dadurch gekennzeichnet, dass als Katalysator Di-n-butylamin eingesetzt wird.

**Claims**

1. A process for producing compounds of the formula I

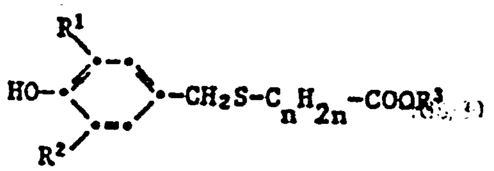

(I)

wherein $R^1$ and $R^2$ independently of one another are each tert-butyl, $R^3$ is $C_1$-$C_{20}$-alkyl, or $C_2$-$C_{20}$-alkyl interrupte by -O- or -S-, and n is the number 1 or 2, by reaction of a phenol of the formula II

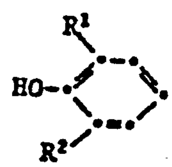

(II)

with formaldehyde and a mercaptan of the formula III

$R^3OOC$-$C_nH_{2n}$-SH (III)

wherein $R^1$, $R^2$, $R^3$ and n have the meanings defined above, at temperatures between 20 and 200°C and in the presence of a catalyst, in which process the catalyst used is a primary or secondary amine of the formula IV

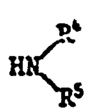

(IV)

in which $R^4$ and $R^5$ independently of one another are each $C_2$-$C_{20}$-alkyl or hydroxyalkyl, $C_5$-$C_7$-cycloalkyl, phenyl or benzyl, and $R^5$ can additionally be hydrogen.

2. A process according to claim 1 for producing compounds of the formula I wherein $R^1$ and $R^2$ independently of one another are each tert-butyl, and $R^3$ is $C_4$-$C_{13}$-alkyl.

3. A process according to claim 2 for producing compounds of the formula I wherein n is the number 1.

4. A process according to claim 1 for producing 3,5-di-tert-butyl-4-hydroxybenzylmercaptoacetic acid-2-ethyl-n-hexyl ester.

5. A process according to claim 1 for producing 3,5-di-tert-butyl-4-hydroxybenzylmercaptoacetic acid-isotridecyl ester.

6. A process according to claim 1, which process comprises reacting 1 mol of the phenol of the formula II with 1.0 to 2.0 mol of formaldehyde and 0.9 to 1.3 mol of a mercaptan of the formula III in the presence of at least 0.5 mol %, relative to the phenol of the formula II, of the catalyst of the formula IV.

7. A process according to claim 1, wherein there is used a catalyst of the formula IV in which $R^4$ and $R^5$ are $C_2$-$C_8$-alkyl, and $R^5$ can additionally be hydrogen.

8. A process according to claim 1, wherein the catalyst used is di-n-butylamine.

## Revendications

1. Procédé pour préparer des composés répondant à la formule I:

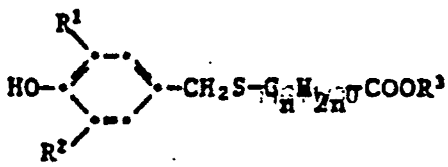 (I)

dans laquelle $R^1$ et $R^2$ représentent, indépendamment l'un de l'autre, un radical tert-butyle, $R^3$ représente un alkyle en $C_1$-$C_{20}$ ou un alkyle en $C_2$-$C_{20}$ interrompu par -O- ou -S- et n désigne un nombre égal à 1 ou à 2, par réaction d'un phénol de formule II:

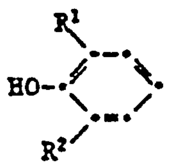 (II)

avec le formaldéhyde et un mercaptan de formule III:

$R^3OOC$-$C_nH_{2n}$-SH (III)

formules dans lesquelles $R^1$, $R^2$, $R^3$ et n ont les significations indiquées plus haut, à des températures comprises entre 20 et 200°C et en présence de catalyseurs, procédé caractérisé en ce que le catalyseur est une amine primaire ou secondaire répondant à la formule IV:

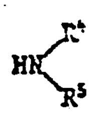 (IV)

dans laquelle $R^4$ et $R^5$ représentent chacun, indépendamment l'un de l'autre, un alkyle en $C_2$-$C_{20}$, un hydroxyalkyle en $C_2$-$C_{20}$, un cycloalkyle en $C_5$-$C_7$, un phényle ou un benzyle, et $R^5$ peut en outre représenter l'hydrogène.

2. Procédé selon la revendication 1 pour la préparation de composés de formule I dans lesquels $R^1$ et $R^2$ représentent chacun un radical tert-butyle et $R^3$ représente un radical alkyle en $C_4$-$C_{13}$.

3. Procédé selon la revendication 2 pour la préparation de composés de formule I dans lesquels n est égal à 1.

4. Procédé selon la revendication 1 pour la préparation du (di-tert-butyl-3,5 hydroxy-4 benzylthio)-acétate d'éthyl-2 hexyle.

5. Procédé selon la revendication 1 pour la préparation du (di-tert-butyl-3,5 hydroxy-4 benzylthio)-acétate d'isotridécyle.

6. Procédé selon la revendication 1 caractérisé en ce qu'on fait réagir 1 mol du phénol de formule II avec de 1,0 à 2,0 mol de formaldéhyde et de 0,9 à 1,3 mol d'un mercaptan de formule III, en présence d'au moins 0,5 % en moles, par rapport au phénol de formule II, du catalyseur de formule IV.

7. Procédé selon la revendication 1 caractérisé en ce qu'on utilise un catalyseur de formule IV dans lequel $R^4$ et $R^5$ représentent chacun un alkyle en $C_2$-$C_8$ et $R^5$ peut en outre représenter l'hydrogène.

8. Procédé selon la revendication 1 caractérisé en ce qu'on utilise la di-n-butylamine comme catalyseur.